# FASCICULE DE BREVET EUROPEEN

(11) **EP 1 460 953 B1**
(45) Date de publication et mention de la délivrance du brevet: **27.04.2005**
(21) Numéro de dépôt: 02801074.2
(22) Date de dépôt: 22.11.2002
(51) Int. Cl.: A61B 17/80

(54) **IMPLANT ORTHOPEDIQUE CONSTITUE D UNE STRUCTURE SUPPORT MUNIE D AUMOINS UN ORIFICE POUR LE PASSAGE D UNE VIS DE FIXA TION ASSOCIEE A UN ECROU**
ORTHOPÄDISCHES IMPLANTAT AUS EINER STÜTZKONSTRUKTION MIT MINDESTENS EINER ÖFFNUNG ZUM DURCHFÜHREN EINER FIXIERSCHRAUBE MIT EINER MUTTER
ORTHOPEDIC IMPLANT CONSISTING OF A SUPPORT STRUCTURE PROVIDED WITH AT LEAST AN ORIFICE FOR PASSING THROUGH A FIXING SCREW ASSOCIATED WITH A NUT

(30) Priorité: 22.11.2001 FR 0115116
(43) Date de publication de la demande: 29.09.2004
(73) Titulaire: D.L.P., 44800 Saint Herblain (FR)
(72) Inventeur: DEROUET, Guillaume, F-44000 Nantes (FR)
(74) Mandataire: Catherine, Alain
(86) Numéro de dépôt international: PCT/FR2002/004020
(87) Numéro de publication internationale: WO 2003/043513

(56) Documents cités:
- WO-A-94/16634
- WO-A-99/09903
- US-A- 5 269 784
- US-A- 5 607 428
- US-A1- 2001 014 807

## Description

La présente invention concerne un nouveau système d'implant orthopédique du type constitué d'une structure support munie d'au moins un orifice pour le passage d'une vis de fixation associée à un écrou.

D'une manière générale, les implants orthopédiques, en particulier les implants d'ostéosynthèse, comprennent une structure support en forme de plaque, coque ou autre, munie d'un ou de plusieurs orifices destinés au passage de vis de fixation aptes à venir s'ancrer dans le matériau osseux de réception.
La forme générale et les dimensions de cette structure support sont adaptées aux contraintes d'implantation.

Il existe de très nombreux types d'implants qui se différencient en particulier par la présence ou non d'une solidarisation entre la tête de vis et la structure support, pour optimiser le maintien en place de la vis dans le temps, et/ou par la présence de moyens conférant à la vis de fixation une possibilité d'orientation angulaire par rapport à l'axe de son orifice de réception, pour permettre au praticien de positionner au mieux ladite vis en fonction du site d'implantation et des contraintes spatiales correspondantes.

On obtient une bonne qualité de maintien de l'implant lorsque la structure support est prise en sandwich entre la tête de vis et un écrou, comme cela est connu du document WO-A-99/09903. Mais dans ce cas la solidarisation particulière de l'écrou avec la structure support nécessite une présentation précise des vis de fixation pour ne pas abîmer le filetage de l'écrou au moment du vissage ; et le praticien ne dispose d'aucune possibilité d'orientation spatiale des vis.

La présente invention propose un nouveau dispositif implantable orthopédique, de structure simple, pratique, disposant de bonnes qualités de blocage et de maintien en place, et autorisant une certaine possibilité d'orientation spatiale des vis de fixation par rapport à l'axe des orifices de réception aménagés dans la structure support.

Cet implant orthopédique est du type constitué d'une structure support munie d'au moins un orifice pour le passage d'une vis de fixation associée à un écrou, la tête de ladite vis étant destinée à venir prendre appui d'un côté dudit support, et ledit écrou étant destiné à venir prendre appui de l'autre côté dudit support, dans un logement qui permet son intégration au moins partielle, de manière à permettre un serrage de ladite structure support entre la tête de vis et l'écrou, en fin de serrage du corps de vis dans le matériau osseux de réception ; ce dispositif implantable comprend également des moyens pour maintenir ledit écrou dans son logement de réception en regard de l'orifice de la structure support, et pour bloquer ledit écrou en rotation. Un tel implant est connu du document WO-A-94/16634.
Conformément à la présente invention, les contours du logement de la structure support et les contours de l'écrou sont dimensionnés pour procurer au moins un degré de liberté audit écrou dans ledit logement, permettant ainsi un autocentrage de la vis de fixation et de l'écrou associé, quelle que soit l'orientation admissible de l'axe de ladite vis par rapport à l'axe de l'orifice de la structure support, et les surfaces de contact écrou (10)/structure support (1) et tête de vis (6)/structure support (1) sont adaptées pour obtenir un blocage correct de ladite structure support (1) entre ladite tête de vis (6) et ledit écrou (10), en fin de serrage, quelle que soit l'orientation de vis choisie.

Selon une première forme de réalisation possible, les moyens de maintien de l'écrou dans son logement de réception sont constitués par un refoulement de matière venant fermer partiellement ledit logement.

Selon un autre mode de réalisation, ces moyens de maintien sont constitués d'un clip rapporté venant fermer partiellement le logement de réception d'écrou.
Dans ce dernier mode de réalisation, le clip de maintien d'écrou se présente avantageusement sous la forme d'une boucle circulaire ouverte ; cette boucle comporte une nervure d'encliquetage qui coopère avec une gorge de forme appropriée aménagée dans le logement d'intégration de l'écrou.

Selon une autre caractéristique, les moyens pour bloquer l'écrou en rotation dans le logement d'accueil de la structure support sont constitués d'au moins un relief aménagé sur l'une desdites pièces (écrou ou structure support), coopérant avec une encoche adaptée, aménagée sur l'autre pièce (structure support ou écrou).

Selon un mode de réalisation particulier, l'implant comporte des surfaces de contact cylindriques entre la tête de vis et la structure support, d'une part, et entre la structure support et l'écrou, d'autre part. Ces surfaces de contact cylindriques sont adaptées pour conférer à la vis de fixation une possibilité d'orientation par rapport à la structure support, tout en conservant.des contacts entre surfaces (contacts cylindriques) permettant d'optimiser la liaison lors du serrage.
De préférence ces surfaces cylindriques de contact ont le même axe.

Selon un mode de réalisation préféré, l'implant comporte des surfaces de contact sphériques entre la tête de vis et la structure support, d'une part, et entre la structure support et l'écrou, d'autre part, qui sont adaptées pour conférer à la vis de fixation une possibilité d'orientation par rapport à la structure support, tout en conservant des contacts de surfaces (contacts sphériques). Cette particularité confère d'importantes possibilités de réglage angulaire de la vis et optimise la liaison mécanique lors du serrage. De préférence ces surfaces de contact sphériques, en forme de calottes sphériques ont le même centre.

Pour obtenir un ensemble compact et ayant une capacité intéressante de réglage angulaire des vis, l'axe ou le centre des surfaces de contact cylindriques ou sphériques, est situé à proximité du plan supérieur de la structure support, ou est confondu avec ce plan.

Selon encore une autre particularité, l'implant orthopédique conforme à l'invention comporte un écrou comprenant un fût cylindrique présentant un filetage femelle, et une calotte ou couronne sphérique située à la périphérie dudit fût cylindrique.

Selon encore une autre particularité, la vis de fixation est munie d'un filetage de corps apte à coopérer avec le matériau osseux de réception, et d'un filetage de tête apte à coopérer avec l'écrou associé, le diamètre externe du filetage de corps étant inférieur ou égal au diamètre externe du filetage de tête pour permettre le passage de la vis à travers l'écrou.

Pour optimiser le serrage, la ou les vis de fixation sont avantageusement munies d'un filetage de tête composé de n filets décalés de 1/n tours, et dont le pas coopère avec celui du filetage de l'écrou et à celui du filetage de corps.

L'invention sera encore illustrée, sans y être aucunement limitée, par la description suivante de plusieurs modes de réalisation particuliers, donnés uniquement à titre d'exemples et représentés sur les dessins annexés dans lesquels :
- la figure 1 représente de façon générale une plaque orthopédique positionnée à la surface d'un os fracturé, et destinée à recevoir des vis de fixation ;
- la figure 2 est un synoptique fonctionnel d'une première forme de réalisation possible d'un implant conforme à la présente invention ;
- la figure 3 illustre en vue de dessus une forme de réalisation possible d'un orifice aménagé dans la structure support pour le passage d'une vis de fixation ;
- les figures 4 à 7 illustrent quatre synoptiques fonctionnels différents d'implants conformes à la présente invention, autorisant des réglages angulaires de l'axe de la vis par rapport à la structure support ;
- la figure 8 est une vue en coupe, éclatée, d'une forme de réalisation préférée d'implant conforme au synoptique fonctionnel de la figure 7 ;
- la figure 9 montre l'implant de la figure 8, en fin de vissage droit, c'est-à-dire que la vis est centrée sur l'axe de l'orifice du support ;
- la figure 10 montre le même implant que figure 9 mais en fin de vissage incliné, c'est-à-dire que la vis fait un angle avec l'axe de l'orifice du support ;
- la figure 11 est une vue partielle à grande échelle, du dessous de la structure support montrant le logement de réception de l'écrou ;
- la figure 12 est une vue en coupe selon 12-12 de la figure 11 ;
- la figure 13 est une perspective à grande échelle également, d'un écrou pour l'implant illustré sur les figures 8 à 10 ;
- la figure 14 est une vue de dessus de l'écrou illustré sur la figure 13 ;
- la figure 15 est une vue en coupe diamétrale selon 15-15 de la figure 14 ;
- la figure 16 est une vue de côté de l'écrou illustré sur les figures 13 à 15 ;
- la figure 17 est une perspective à grande échelle du clip circulaire permettant le maintien de l'écrou dans le logement de la structure support ;
- la figure 18 est une vue de dessus du clip illustré sur la figure 17 ;
- la figure 19 est une vue en coupe diamétrale du clip selon 19-19 de la figure 18.

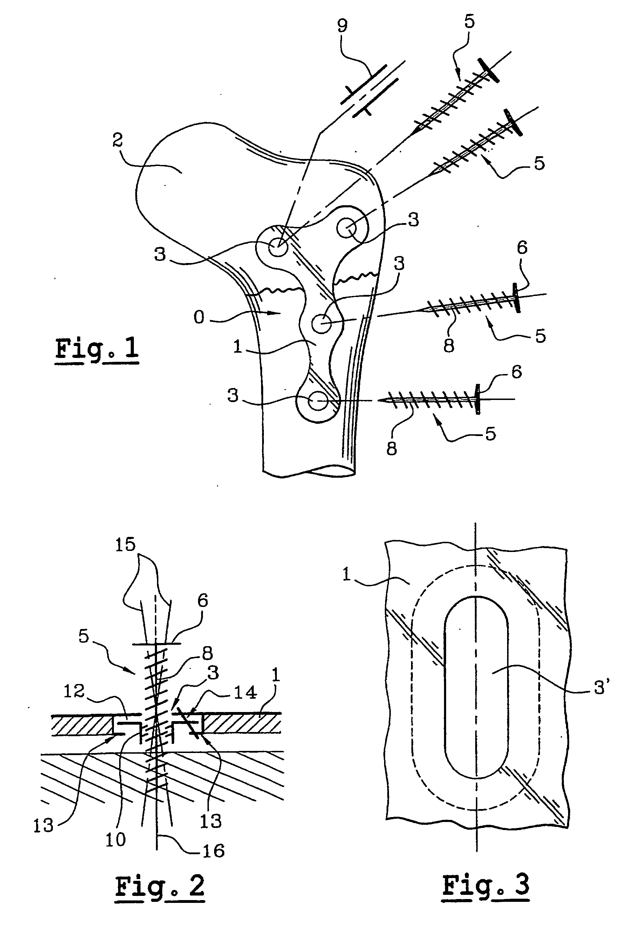

Sur la figure 1 on remarque l'implant orthopédique 0 constitué d'une structure support en forme de plaque d'ostéosynthèse 1 positionnée sur un os fracturé 2, par exemple une épiphyse du radius, et présentant quatre orifices circulaires 3 pour la réception de vis de fixation 5. Chaque vis 5 comprend une tête de vis 6 et un corps de vis 8.

Sur la figure 1, on a également illustré le canon de perçage 9 qui est utilisé avant la mise en place des vis 5 pour percer des trous de positionnement dans le matériau osseux, à travers les orifices 3.

La figure 2 est un synoptique fonctionnel qui montre schématiquement le montage d'une vis 5 sur la plaque support 1.

Sur cette figure, on remarque la présence d'un écrou 10 positionné dans un logement 12 aménagé dans la plaque support 1, de manière à ce qu'en fin de vissage ladite plaque 1 soit prise en sandwich entre la tête 6 de la vis 5 et ledit écrou 10. Le filetage femelle de l'écrou 10 coopère avec le filetage mâle du corps de vis 8 pour réaliser un assemblage bloqué en fin de vissage.
A cet effet, la vis 5 peut comporter un filetage unique, d'une part pour l'ancrage dans le matériau osseux, et d'autre part pour la coopération avec l'écrou ; mais elle peut aussi être munie de deux filetages différents assurant chacune l'une des fonctions précitées.

L'écrou 10 peut être simplement partiellement intégré dans le logement 12. Il est maintenu en place dans ledit logement 12, en regard de l'orifice 3, par des moyens de maintien appropriés 13, et il est immobilisé en rotation également par des moyens appropriés représentés sous la forme d'un simple trait repéré 14.

Les moyens de maintien 13 peuvent se présenter sous la forme d'un refoulement de matière ou d'une structure rapportée détaillée plus loin, du type clip de verrouillage, mis en oeuvre après le positionnement de l'écrou 10 dans le logement 12.

Le blocage en rotation de l'écrou 10 est obtenu soit par les formes générales relatives de l'écrou 10 et du logement 12 de réception, ou par coopération d'organes complémentaires du type relief(s) et encoche(s), aménagés sur les surfaces en regard de l'écrou et du logement.

Conformément à l'invention, les contours du logement 12 et les contours de l'écrou 10 sont adaptés et dimensionnés pour procurer à ce dernier, dans ledit logement, au moins un degré de liberté, supérieur à celui d'un simple jeu fonctionnel, permettant ainsi un autocentrage de la vis 5 et de l'écrou 10, ceci quelle que soit l'orientation admissible de l'axe de ladite vis par rapport à l'axe du logement 3.
Sur la figure 2, on remarque les deux traits d'axes 15 illustrant l'amplitude des inclinaisons correspondantes possibles de la vis. La position médiane, normale à la plaque support 1 est représentée par l'axe 16, lequel axe 16 correspond à l'axe du logement 3.

Comme représenté sur la figure 3, la plaque support 1 peut comporter des orifices allongés 3' autorisant alors une possibilité de réglage longitudinal supplémentaire des vis 5 par rapport au support 1. Ces orifices allongés 3' peuvent présenter une forme générale rectiligne ou curviligne.

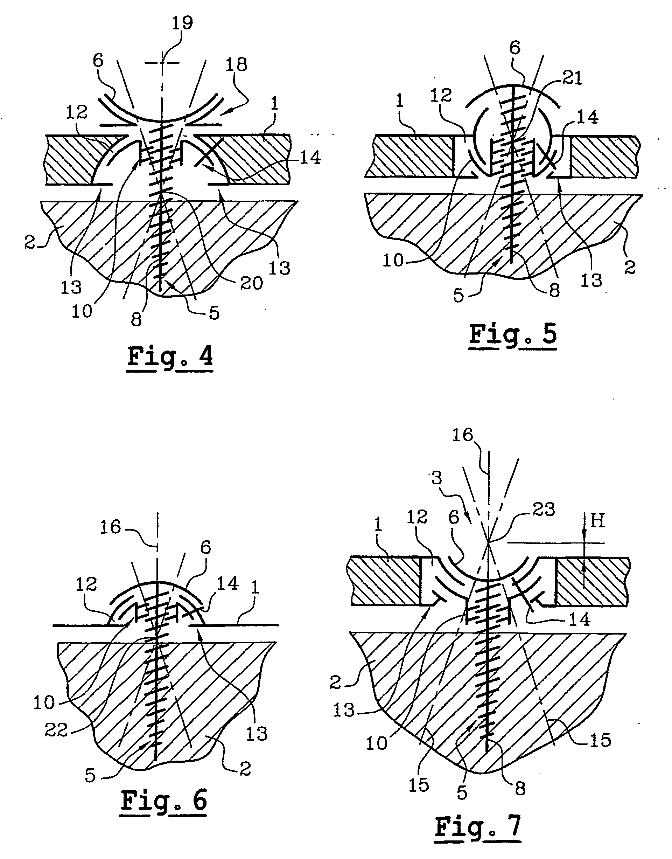

Les figures 4, 5, 6 et 7 sont des schémas fonctionnels dérivés de celui de la figure 2, illustrant le montage vissé conforme à l'invention, mais avec des surfaces de contact cylindriques ou sphériques entre la tête de vis 6 et la plaque support 1, d'une part, et entre la plaque support 1 et l'écrou 10, d'autre part, autorisant un degré de liberté et des possibilités de réglage angulaire de la vis 5 relativement importantes, tout en conservant une surface de contact importante favorisant la bonne cohésion de l'ensemble après vissage.
Dans tous les cas, les différentes pièces sont conformées au mieux pour obtenir des possibilités de réglage angulaire importantes et pour conserver les meilleurs contacts entre surfaces possibles. Dans le cas de surfaces de contact cylindriques, le réglage angulaire est possible dans un plan si l'orifice 3 est circulaire, ou dans différents plans parallèles correspondant à un volume prismatique si on dispose d'un orifice 3' oblong tel qu'illustré sur la figure 3.
Dans le cas de surfaces de contact sphériques, on prévoit de préférence un orifice d'accueil circulaire et le réglage angulaire de la vis est alors possible à l'intérieur d'un volume conique dont l'axe est confondu avec celui de l'orifice circulaire 3.

Sur la figure 4, la tête 6 de la vis 5 est en contact avec un organe intermédiaire 18 qui comporte une surface de glissement en contact avec la plaque support 1. Les surfaces de contact entre la tête 6 et cet organe rapporté 18 correspondent à une portion cylindrique centrée sur un axe 19, ou à une calotte sphérique centrée en un point 19, selon le cas. L'axe ou le centre 19 est ici positionné côté extérieur, au-dessus de la plaque support 1.
D'autre part, l'écrou 10 et la plaque support 1 sont également en contact sur des surfaces cylindriques ou sphériques, selon le cas. L'axe ou le centre 20 de la portion de cylindre ou de la portion de calotte sphérique correspondante est ici positionné côté intérieur, c'est-à-dire du côté de la structure osseuse.

L'écrou 10 est maintenu en place par les moyens 13 et il est immobilisé en rotation par des moyens 14 schématisés sous la forme d'un simple trait.

Les figures 5, 6 et 7 illustrent des modes de réalisation dépourvus de pièce complémentaire 18 et dont les surfaces de contact sphériques ou cylindriques tête de vis/plaque support et plaque support/écrou sont centrées sur le même axe ou présentent le même centre.

Pour le mode de réalisation illustré sur la figure 5, l'axe ou le centre correspondant 21 est situé entre les deux surfaces de contact.

Pour le mode de réalisation illustré sur la figure 6, l'axe ou le centre correspondant 22 est situé côté face interne de la structure support 1.

Pour le mode de réalisation de la figure 7, l'axe ou le centre correspondant 23 est situé côté face externe de la structure support 1.

Le mode de réalisation illustré sur la figure 7 apparaît le plus compact. De préférence l'axe ou le centre 23 est situé à proximité de la surface supérieure de la structure support 1 pour optimiser les possibilités de débattement angulaire de la vis 5 ; la distance repérée H sur cette figure tend alors vers une valeur nulle.

Dans tous les cas, l'écrou 10 est maintenu dans son logement 12 par des moyens 13 du genre refoulement de matière ou clip comme détaillé plus loin, et il est immobilisé en rotation par des moyens représentés schématiquement sous la forme d'un simple trait 14 détaillé plus loin également.

Du fait de l'encombrement général des pièces, le cône correspondant de débattement angulaire confère toujours au praticien des possibilités intéressantes de réglage.

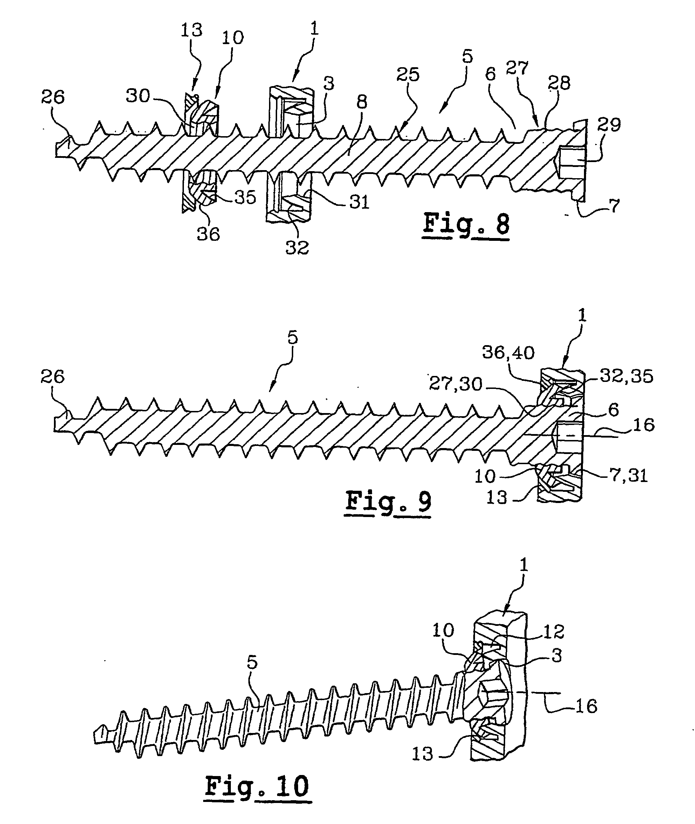

Les figures 8 à 19 détaillent un mode de réalisation conforme à celui représenté schématiquement sur la figure 7.

La figure 8 représente une vis de fixation 5 en coupe schématique longitudinale. Cette vis 5 présente d'une part un corps de vis 8 muni d'un filetage 25 et dont l'extrémité 26 est conformée en queue de cochon, et d'autre part une tête de vis 6 munie d'un filetage propre 27. La partie supérieure de la tête de vis 6 présente une cavité 29 à parois polygonales coopérant avec l'ancillaire de pose.

Cette tête de vis 6 présente à son extrémité une collerette dont le contour périphérique est en forme de couronne sphérique 7, destinée au contact avec une surface de forme équivalente aménagée sur la structure support 1.

La vis 5 traverse la plaque 1, vue en coupe, au niveau de l'orifice circulaire 3, ainsi que l'écrou 10 et les moyens de maintien 13, ici en forme de clip de verrouillage, également vus en coupe.

La structure support 1, l'écrou 10 et le clip de verrouillage 13 sont représentés éclatés, séparés les uns des autres.

Le filetage 27 de la tête de vis 5 est adapté à celui de l'écrou 10. Ce filetage est composé de n filets 28 décalés de 1/n tour, dont le pas est apte à coopérer avec celui du filetage 30 de l'écrou 10, lequel pas correspond à celui du filetage 25 du corps de vis 8.

Le filetage 25 du corps de vis 8 est apte à venir réaliser un ancrage par vissage dans le matériau osseux ; le diamètre externe de ce filetage de corps 25 est inférieur ou égal au diamètre externe du filetage de tête 27 de manière à permettre le passage du corps de vis 8 au travers de l'orifice fileté de l'écrou 10.

La figure 9 représente la vis de fixation 5 en fin de vissage, dans une configuration normale à la plaque 1 (la structure osseuse de réception n'est pas représentée). La plaque 1 se trouve alors prise en sandwich entre d'une part la tête de vis 6, et d'autre part l'écrou 10 dont le filetage femelle 30 coopère avec le filetage mâle 27 de ladite tête de vis 6.

La figure 10 représente la vis de fixation 5 en fin de vissage dans une configuration inclinée par rapport à l'axe 16 de l'orifice 3. Cette possibilité d'inclinaison est liée au fait que l'écrou 10 dispose d'un degré de liberté dans son logement de réception 12. D'autre part, les surfaces de contact sphériques tête de vis/plaque support et plaque support/écrou permettent d'obtenir une grande qualité de serrage quelle que soit l'inclinaison admissible de l'axe de la vis 5 par rapport à l'axe 16 de l'orifice 3.

On peut prévoir également une surface de contact sphérique entre l'écrou 10 et le clip de verrouillage 13 de manière à optimiser le guidage de l'écrou 10 lors du positionnement de la vis 5 en début de vissage.

Les surfaces de contact correspondantes sont détaillées plus loin en liaison avec la description de chaque pièce constitutive de l'implant.

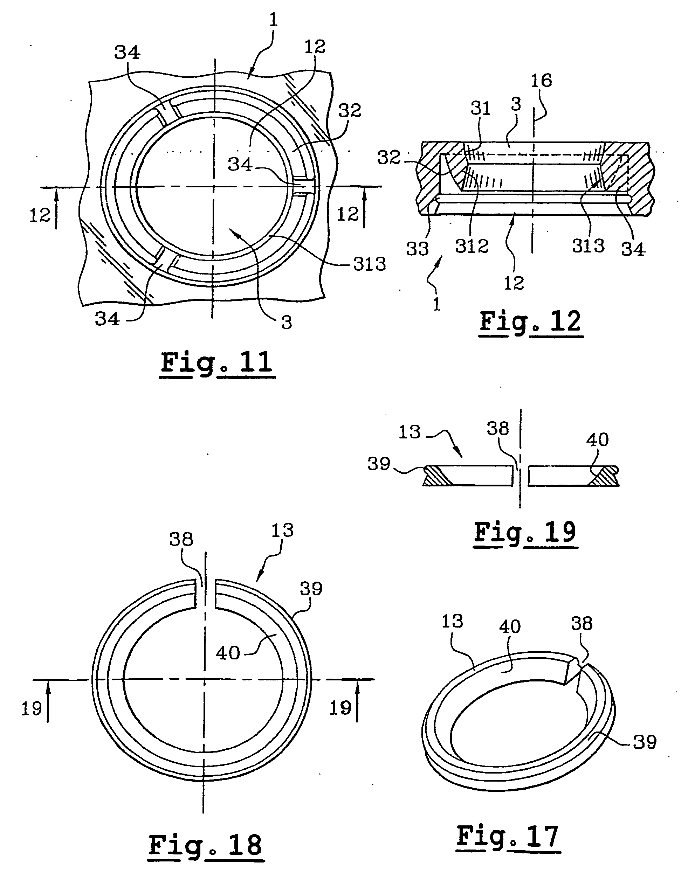

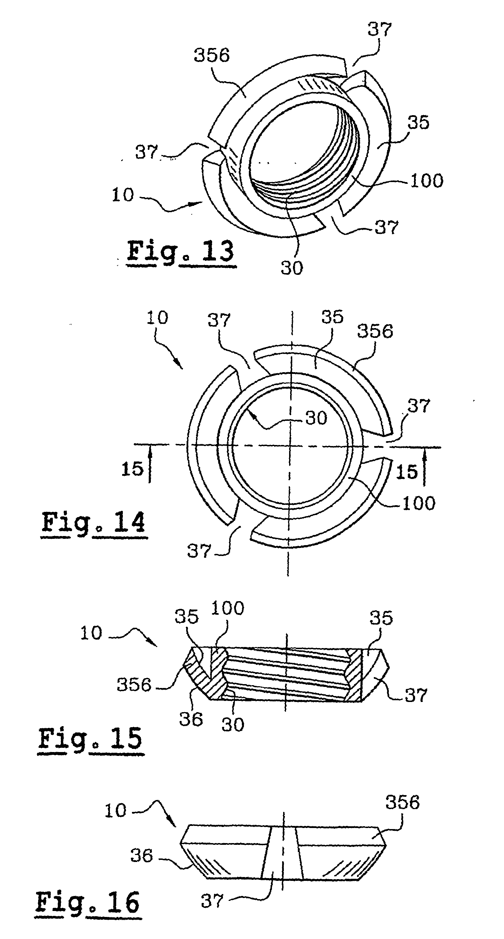

Les figures 11 et 12 représentent en détails la configuration de l'orifice circulaire 3 et du logement 12 qui est aménagé dans la structure support 1, lequel logement 12 est destiné à recevoir l'écrou 10 détaillé sur les figures 13 à 16. Cet écrou 10 est de préférence maintenu par un clip de verrouillage détaillé figures 17 à 19, lequel clip permet un démontage plus facile de l'écrou que dans le cas d'un maintien par sertissage.

Telle que représentée sur les figures 11 et 12, la plaque support 1 comporte un anneau sphérique 312 constitué d'une première couronne sphérique 31 pour le contact avec la tête de vis 6, et d'une seconde couronne sphérique 32 pour le contact avec l'écrou 10.

La portion périphérique 313 qui s'étend entre les deux couronnes sphériques 31 et 32, est de forme tronconique, avec un angle suffisant pour permettre le mouvement de l'écrou 10 et donc de la vis 5 par rapport à la pièce support 1. Cette portion 313 peut constituer une butée pour l'écrou 10 et en particulier pour son fût 100 comme détaillé plus loin.

Sur la périphérie circulaire du logement 12, on remarque la présence d'une gorge circulaire 33 destinée au positionnement et au verrouillage du clip de maintien 13.

Les moyens d'immobilisation en rotation de l'écrou 10 par rapport au support 1 sont constitués d'au moins un ergot ou tenon. La figure 11 montre trois tenons 34 régulièrement répartis dans le fond du logement circulaire 12, entre la périphérie dudit logement et la couronne sphérique 32. Ces tenons 34 coopèrent avec des formes homologues femelles détaillées ci-après, aménagées sur l'écrou 10 pour verrouiller ce dernier en rotation.

Tel que représenté sur les figures 13 à 16, l'écrou 10 présente une couronne sphérique 35 destinée au contact avec la structure support 1 et en particulier la couronne sphérique 32 de l'anneau 312. On peut également prévoir une couronne sphérique externe 36 destinée au contact avec le clip de maintien 13. Lesdites couronnes sphériques 35, 36 font partie d'un anneau sphérique 356 relié à la périphérie inférieure d'un fût cylindrique 100 faisant office d'écrou, lequel fût est muni du filetage femelle 30.

La surface périphérique externe du fût 100 se loge avec du jeu dans la portion tronconique 313 du support 1, pour ne pas altérer les contacts sphériques rotuliens entre l'écrou 10 et ledit support.

La limitation de l'amplitude du mouvement de l'écrou 10 par rapport au support 1 peut être obtenue par la portion tronconique 313 dudit support, qui fait office de butée pour le fût 100 de l'écrou 10.

La surface sphérique 36 optimise le positionnement spatial correct de l'écrou au moment de l'introduction de la vis 5. Le degré de liberté de l'écrou 10 dans son logement de réception 12 permet un autocentrage de la vis de fixation 5 et de l'écrou associé au moment de la fixation de l'implant,

Sur les figures 13 et 14, on remarque les formes femelles dont on a parlé plus haut, en forme de cavités ou encoches 37 qui sont destinées à coopérer avec les tenons 34 de la structure support 1 pour verrouiller l'écrou 10 en rotation. Les côtés de ces cavités ou encoches 37 peuvent être parallèles ou être légèrement divergentes, comme illustré sur les figures 13, 14 et 15.
Dans l'exemple de réalisation illustré, les tenons 34 et les encoches homologues 37 sont au nombre de trois, disposés à 120° respectivement dans le fond du logement 12 de la structure support 1, et en périphérie de l'écrou 10. La découpe de la couronne sphérique 356 par les encoches 37 confère une certaine élasticité à l'écrou 10.

Tel que représenté sur les figures 17 à 19, le clip 13 est en forme d'anneau circulaire muni d'une fente 38 lui conférant une certaine élasticité radiale. Sur sa périphérie externe, ce clip 13 est pourvu d'une nervure ou bossage périphérique 39 apte à venir s'encastrer dans la gorge circulaire 33 du logement 12 aménagé dans le support 1. L'encastrement du clip 13 sur la structure support 1 est rendu possible par la présence de la fente 38. Le clip 13 est dimensionné pour assurer le maintien de l'écrou 10 dans le logement 12.
Le clip 13 est également muni intérieurement d'une couronne sphérique 40 apte à coopérer avec la couronne sphérique correspondante 36 aménagée sur l'écrou 10.

Comme on l'a précisé auparavant, en fin de vissage de la vis de fixation 5, les différentes couronnes sphériques : - 7 sur la tête 6 de la vis 5, - 31 et 32 sur le support 1, et - 35 et 36 sur l'écrou 10, sont concentriques ; une fois l'ensemble assemblé, le centre correspondant 23 est positionné sensiblement dans le plan de la surface supérieure de la plaque support 1, tel que cela apparaît sur la figure 9.

## Revendications

1. Dispositif orthopédique implantable constitué d'une structure support (1), d'au moins une vis de fixation (5) et d'un écrou (10), laquelle structure support (1) est munie d'au moins un orifice (3) pour le passage de la vis de fixation (5) associée audit écrou (10), la tête (6) de ladite vis (5) étant destinée à venir prendre appui d'un côté dudit support (1), et ledit écrou (10) étant destiné à venir prendre appui de l'autre côté dudit support (1), dans un logement (12) qui permet son intégration au moins partielle, de manière à permettre un serrage de ladite structure support (1) entre la tête (6) de la vis (5) et l'écrou (10) en fin de vissage du corps de vis dans le matériau osseux de réception, lequel dispositif implantable comprend des moyens pour maintenir ledit écrou (10) dans son logement de réception (12) en regard de l'orifice (3) de la structure support (1), et des moyens pour bloquer ledit écrou (10) en rotation, **caractérisé en ce que** les contours du logement (12) de la structure support (1) et les contours de l'écrou (10) sont dimensionnés pour procurer au moins un degré de liberté audit écrou (10), lorsqu'il est intégré et maintenu dans son logement (12), cela de façon à permettre un auto-centrage de l'écrou (10) par rapport à la vis de fixation (5) associée, quelle que soit l'orientation admissible de l'axe de ladite vis (5) par rapport à l'axe (16) de l'orifice (3) de la structure support (1), lors de la présentation de ladite vis (5) en regard dudit écrou (10) intégré dans son logement (12), et cela de façon à permettre également le choix d'une orientation spatiale précise de ladite vis (5) par rapport à l'axe (16) de l'orifice (3), dans une amplitude d'orientation admissible, lors de son vissage dans le matériau osseux de réception, les surfaces de contact écrou (10)/structure support (1) et tête de vis (6)/structure support (1) étant adaptées pour obtenir un blocage correct de ladite structure support (1) entre ladite tête de vis (6) et ledit écrou (10), en fin de serrage, quelle que soit l'orientation de vis choisie.

2. Dispositif implantable selon la revendication 1, **caractérisé en ce que** les moyens de maintien (13) de l'écrou (10) dans son logement de réception (12) sont constitués par un refoulement de matière venant fermer partiellement ledit logement (12).

3. Dispositif implantable selon la revendication 1, **caractérisé en ce que** les moyens de maintien de l'écrou (10) dans son logement (12) sont constitués d'un clip rapporté (13) venant fermer partiellement ledit logement (12).

4. Dispositif implantable selon la revendication 3, **caractérisé en ce qu'**il comporte un clip (13) de maintien d'écrou (10) en forme de boucle circulaire ouverte, laquelle boucle (13) comporte une nervure d'encliquetage (39) coopérant avec une gorge (33) de forme appropriée aménagée dans le logement (12) d'Intégration de l'écrou (10).

5. Dispositif implantable selon l'une quelconque des revendications 1 à 4, **caractérisé en ce que** les moyens (14) pour bloquer l'écrou (10) un rotation dans le logement d'accueil (12) de la structure support (1) sont constitués d'au moins un relief (34) aménagé sur l'une desdites pièces (1, 10), coopérant avec une encoche (37) adaptée aménagée sur l'autre desdites pièces (10, 1).

6. Dispositif implantable selon l'une quelconque des revendications 1 à 5, **caractérisé en ce qu'**il comporte des surfaces de contact cylindriques entre la tête de vis (6) et la structure support (1), d'une part, et des surfaces de contact cylindriques entre la structure support (1) et l'écrou (10), d'autre part, adaptées pour conférer à la vis de fixation (5) une possibilité d'orientation par rapport à la structure support (1), tout en conservant des contacts entre lesdites surfaces.

7. Dispositif implantable selon la revendication 6, **caractérisé en ce qu'**il comporte des surfaces de contact cylindriques entre la tête de vis (6) et la structure support (1), d'une part, et des surfaces de contact cylindriques entre la structure support (1) et l'écrou (10), d'autre part, lesquelles surfaces ont le même axe (21, 22, 23).

8. Dispositif implantable selon l'une quelconque des revendications 1 à 5, **caractérisé en ce qu'**il comporte des surfaces de contact sphériques (7, 31) entre la tête de vis (6) et la structure support (1), d'une part, et des surfaces de contact cylindriques (32, 35) entre la structure support (1) et l'écrou (10), d'autre part, lesquelles surfaces sont adaptées pour conférer à la vis de fixation (5) une possibilité d'orientation par rapport à la structure support (1), tout en conservant des contacts entre surfaces.

9. Dispositif implantable selon la revendication 8, **caractérisé en ce qu'**il comporte des surfaces de contact sphériques (7, 31 ; 32, 35) entre la tête de vis (6) et la structure support (1), d'une part, et entre la structure support (1) et récrou (10), d'autre part, qui ont le même centre (21, 22, 23).

10. Dispositif implantable selon l'une quelconque des revendications 7 ou 9, **caractérisé en ce qu'**il comporte des surfaces de contact cylindriques ou sphériques dont la position de l'axe, ou du centre (23) selon le cas, est située à proximité du plan supérieur de la structure support (1), voire confondue avec ce plan.

11. Dispositif implantable selon l'une quelconque des revendications 8 à 10, **caractérisé en ce qu'**il comporte un écrou (10) comprenant un fût cylindrique (100) muni d'un filetage femelle (30), et une couronne sphérique (356) située à la périphérie dudit fût cylindrique (100).

12. Dispositif implantable selon l'une quelconque des revendications 1 à 11, **caractérisé en ce que** la vis de fixation (5) est munie d'un filetage de corps (25) apte à coopérer avec le matériau osseux, et d'un filetage de tête (27), différent, apte à coopérer avec l'écrou (10) associé, le diamètre externe du filetage de corps (25) étant inférieur ou égal au diamètre externe du filetage de tête (27).

13. Dispositif implantable selon la revendication 12, **caractérisé en ce qu'**il comporte au moins une vis de fixation (5) dont le filetage de tête (27) est composé de n filets (28) décalés de 1/n tour, dont le pas correspond à celui du filetage (30) de l'écrou (10) et à celui du filetage de corps (25).

## Patentansprüche

1. Implantierbare orthopädische Vorrichtung, die von einer Stützstruktur (1), mindestens einer Befestigungsschraube (5) und einer Mutter (10) gebildet ist, wobei die Stützstruktur (1) mit mindestens einer Öffnung (3) für den Durchgang der Befestigungsschraube (5), die mit der Mutter (10) verbunden ist, versehen ist, wobei der Kopf (6) der Schraube (5) dazu bestimmt ist, auf einer Seite der Stütze (1) zur Auflage zu gelangen, und wobei die Mutter (10) dazu bestimmt ist, auf der anderen Seite der Stütze (1) in einer Lagerung (12) zur Auflage zu gelangen, die zumindest ihre teilweise Versenkung ermöglicht, so dass ein Festklemmen der Stützstruktur (1) zwischen dem Kopf (6) der Schraube (5) und der Mutter (10) am Ende des Einschraubens des Schraubenkörpers in das Knochenaufnahmematerial möglich ist, wobei die implantierbare Vorrichtung Mittel, um die Mutter (10) in ihrer Aufnahmelagerung (12) gegenüber der Öffnung (3) der Stützstruktur (1) zu halten, und Mittel umfasst, um die Mutter (10) in Bezug auf eine Drehung festzustellen, **dadurch gekennzeichnet, dass** die Konturen der Lagerung (12) der Stützstruktur (1) und die Konturen der Mutter (10) derart dimensioniert sind, dass sie der Mutter (10) zumindest einen Freiheitsgrad verleihen, wenn sie in ihrer Lagerung (12) versenkt und gehalten wird, um eine Selbstzentrierung der Mutter (10) in Bezug auf die zugehörige Befestigungsschraube (5) zu ermöglichen, und zwar unabhängig von der zulässigen Ausrichtung der Achse der Schraube (5) in Bezug auf die Achse (16) der Öffnung (3) der Stützstruktur (1), wenn die Schraube (5) gegenüber der Mutter (10), die in ihrer Lagerung (12) versenkt ist, zur Anordnung gelangt, und auch um die Wahl einer genauen räumlichen Ausrichtung der Schraube (5) in Bezug auf die Achse (16) der Öffnung (3) in einer zulässigen Ausrichtungsamplitude bei ihrem Schrauben in das Knochenaufnahmematerial zu ermöglichen, wobei die Kontaktflächen Mutter (10) / Stützstruktur (1) und Schraubenkopf (6) / Stützstruktur (1) derart ausgeführt sind, dass sie ein richtiges Festlegen der Stützstruktur (1) zwischen dem Schraubenkopf (6) und der Mutter (10) am Ende des Festklemmens erzielen, und zwar unabhängig von der gewählten Ausrichtung der Schraube.

2. Implantierbare Vorrichtung nach Anspruch 1, **dadurch gekennzeichnet, dass** die Mittel (13) zum Halten der Mutter (10) in ihrer Aufnahmelagerung (12) von gestauchtem Material gebildet sind, das die Lagerung (12) teilweise verschließt.

3. Implantierbare Vorrichtung nach Anspruch 1, **dadurch gekennzeichnet, dass** die Mittel zum Halten der Mutter (10) in ihrer Lagerung (12) von einem aufgesetzten Clip (13) gebildet sind, das die Lagerung (12) teilweise verschließt.

4. Implantierbare Vorrichtung nach Anspruch 3, **dadurch gekennzeichnet, dass** sie einen Clip (13) zum Halten der Mutter (10) in Form einer offenen kreisförmigen Schleife umfasst, wobei diese Schleife (13) eine Rastrippe (39) umfasst, die mit einer Rille (33) von geeigneter Form zusammenwirkt, die in der Lagerung (12) zum Versenken der Mutter (10) vorgesehen ist.

5. Implantierbare Vorrichtung nach einem der Ansprüche 1 bis 4, **dadurch gekennzeichnet, dass** die Mittel (14) zum Drehfeststellen der Mutter (10) in der Aufnahmelagerung (12) der Stützstruktur (1) von mindestens einem Vorsprung (34) gebildet sind, der auf einem der genannten Teile (1, 10) vorgesehen ist und mit einer entsprechenden Kerbe (37) zusammenwirkt, die auf dem anderen der Teile (10, 1) vorgesehen ist.

6. Implantierbare Vorrichtung nach einem der Ansprüche 1 bis 5, **dadurch gekennzeichnet, dass** sie zylindrische Kontaktflächen zwischen dem Schraubenkopf (6) und der Stützstruktur (1) einerseits und zylindrische Kontaktflächen zwischen der Stützstruktur (1) und der Mutter (10) andererseits umfasst, die derart ausgeführt sind, dass sie der Befestigungsschraube (5) eine Möglichkeit der Ausrichtung in Bezug auf die Stützstruktur (1) verleihen, wobei sie gleichzeitig Kontakte zwischen den Flächen aufrecht erhalten.

7. Implantierbare Vorrichtung nach Anspruch 6, **dadurch gekennzeichnet, dass** sie zylindrische Kontaktflächen zwischen dem Schraubenkopf (6) und der Stützstruktur (1) einerseits und zylindrische Kontaktflächen zwischen der Stützstruktur (1) und der Mutter (10) andererseits umfasst, wobei diese Flächen dieselbe Achse (21, 22, 23) besitzen.

8. Implantierbare Vorrichtung nach einem der Ansprüche 1 bis 5, **dadurch gekennzeichnet, dass** sie kugelförmige Kontaktflächen (7, 31) zwischen dem Schraubenkopf (6) und der Stützstruktur (1) einerseits und zylindrische Kontaktflächen (32, 35) zwischen der Stützstruktur (1) und der Mutter (10) andererseits umfasst, wobei die Flächen derart ausgeführt sind, dass sie der Befestigungsschraube (5) eine Möglichkeit der Ausrichtung in Bezug auf die Stützstruktur (1) verleihen, wobei sie gleichzeitig Kontakte zwischen den Flächen aufrecht erhalten.

9. Implantierbare Vorrichtung nach Anspruch 8, **dadurch gekennzeichnet, dass** sie kugelförmige Kontaktflächen (7, 31; 32, 35) zwischen dem Schraubenkopf (6) und der Stützstruktur (1) einerseits und zwischen der Stützstruktur (1) und der Mutter (10) andererseits umfasst, die denselben Mittelpunkt (21, 22, 23) besitzen.

10. Implantierbare Vorrichtung nach einem der Ansprüche 7 oder 9, **dadurch gekennzeichnet, dass** sie zylindrische oder kugelförmige Kontaktflächen umfasst, bei denen die Position der Achse oder je nach Fall des Mittelpunktes (23) in der Nähe der oberen Ebene der Stützstruktur (1) angeordnet ist bzw. mit dieser Ebene zusammenfällt.

11. Implantierbare Vorrichtung nach einem der Ansprüche 8 bis 10, **dadurch gekennzeichnet, dass** sie eine Mutter (10) umfasst, die einen zylindrischen Schaft (100), der mit einem weiblichen Gewinde (30) versehen ist, und einen kugelförmigen Kranz (356) besitzt, der sich an der Peripherie des zylindrischen Schafts (100) befindet.

12. Implantierbare Vorrichtung nach einem der Ansprüche 1 bis 11, **dadurch gekennzeichnet, dass** die Befestigungsschraube (5) mit einem Körpergewinde (25), das mit dem Knochenmaterial zusammenwirken kann, und einem hierzu unterschiedlichen Kopfgewinde (27) versehen ist, das mit der zugehörigen Mutter (10) zusammenwirken kann, wobei der Außendurchmesser des Körpergewindes (25) kleiner oder gleich dem Außendurchmesser des Kopfgewindes (27) ist.

13. Implantierbare Vorrichtung nach Anspruch 12, **dadurch gekennzeichnet, dass** sie mindestens eine Befestigungsschraube (5) umfasst, deren Kopfgewinde (27) aus n Gewindegängen (28), die um 1/n Umdrehung versetzt sind und deren Steigung jener des Gewindes (30) der Mutter (10) und jener des Körpergewindes (25) entspricht, besteht.

## Claims

1. An implantable orthopaedic device made of a supporting structure (1) at least a fastening screw (5) and a nut (10), said supporting structure (1) is provided with at least one orifice (3) to let through the fastening screw (5) associated with the nut (10), the head (6) of said screw (5) being intended for resting on one side of said support (1), and said nut (10) being intended for resting on the other side of said support (1), in a housing (12) which enables at least partial integration, to allow clamping said supporting structure (1) between the head (6) of the screw (5) and the nut (10) upon complete screwing of the screw body in the receiving bone material, which implantable device comprises means for holding said nut (10) in its reception housing (12) facing the orifice (3) of the supporting structure (1), and means for locking said nut (10) in rotation, **characterised in that** the contours of the housing (12) of the supporting structure (1) and the contours of the nut (10) are sized to provide at least one degree of freedom to said nut (10) when it is inserted and held in the housing thereof (12) to enable a self-centring of the nut (10) with respect of the associated fastening screw (5) regardless of the admissible orientation of the axis of said screw (5) relative to the axis (16) of the orifice (3) of the supporting structure (1) when the said screw (5) is brought facing said nut (10) inserted in the housing thereof (12) and to enable also an accurate spatial orientation of said screw (5) with respect to the axis (16) of the orifice (3) with an admissible orientation amplitude during screwing in the reception bone material, the contact surfaces : nut (10)/ supporting structure (1) and screw head (6)/ supporting structure (1) being provided to obtain a suitable locking of said supporting structure (1) between said screw head (6) and said nut (10) at the end of the clamping regardless the selected screw orientation.

2. An implantable device according to claim 1, **characterised in that** the holding means (13) of the nut (10) in its reception housing (12) are made of a material rebound closing partially said housing (12).

3. An implantable device according to claim 1, **characterised in that** the holding means of the nut (10) in its housing (12) are made of an added-on clip (13) closing partially said housing (12).

4. An implantable device according to claim 3, **characterised in that** it comprises a clip (13) for holding a nut (10) in the form of an open circular loop, which loop (13) comprises a snap-on groove (39) co-operating with a groove (33) of appropriate shape provided in the integration housing (12) of the nut (10).

5. An implantable device according to any one of claims 1 to 4, **characterised in that** the means (14) for locking the nut (10) in rotation in the reception housing (12) of the supporting structure (1) are made of at least one relief (34) laid out on one of said parts (1, 10), co-operating with an adapted notch (37) provided on the other one of said parts (10, 1).

6. An implantable device according to any one of claims 1 to 5, **characterised in that** it comprises cylindrical contact surfaces between the screw head (6) and the supporting structure (1), on the one hand, and cylindrical contact surfaces between the supporting structure (1) and the nut (10), on the other hand, adapted to provide to the fastening screw (5) a possibility of orientation relative to the supporting structure (1), while keeping contacts between said surfaces.

7. An implantable device according to claim 6, **characterised in that** it comprises cylindrical contact surfaces between the screw head (6) and the supporting structure (1), on the one hand, and cylindrical contact surfaces between the supporting structure (1) and the nut (10), on the other hand, which surfaces have the same axis (21, 22, 23).

8. An implantable device according to any one of claims 1 to 5, **characterised in that** it comprises spherical contact surfaces (7, 31 ) between the screw head (6) and the supporting structure (1), on the one hand, and cylindrical contact surfaces (32, 35) between the supporting structure (1) and the nut (10), on the other hand, which surfaces are adapted to provide to the fastening screw (5) a possibility of orientation relative to the supporting structure (1), while keeping contacts between surfaces.

9. An implantable device according to claim 8, **characterised in that** it comprises spherical contact surfaces (7, 31 ; 32, 35) between the screw head (6) and the supporting structure (1), on the one hand, and between the supporting structure (1) and the nut (10), on the other hand, which have the same centre (21, 22, 23).

10. An implantable device according to any one of claim 7 or 9, **characterised in that** it comprises cylindrical or spherical contact surfaces whereof the position of the axis, or of the centre (23) as the case may be, is situated in the vicinity of the upper plane of the supporting structure (1), let alone confused with this plane.

11. An implantable device according to any one of claims 8 to 10, **characterised in that** it comprises a nut (10) having a cylindrical shaft (100) provided with a female thread (30), and a spherical crown (356) situated at the periphery of said cylindrical shaft (100).

12. An implantable device according to any one of claims 1 to 11, **characterised in that** the fastening screw (5) is provided with a body thread (25) able to co-operate with the bone material, and with a head thread (27), different, able to co-operate with the associated nut (10), the outer diameter of the body thread (25) being smaller than or equal to the outer diameter of the head thread (27).

13. An implantable device according to claim 12, **characterised in that** it comprises at least one fastening screw (5) whereof the head thread (27) is made of n threads (28) offset by 1/n turn, whereof the pitch corresponds to that of the thread (30) of the nut (10) and to that of the body thread (25).
